# FASCICULE DE BREVET EUROPEEN

(11) **EP 3 982 924 B1**
(45) Date de publication et mention de la délivrance du brevet: **26.07.2023**
(21) Numéro de dépôt: 20734655.2
(22) Date de dépôt: 11.06.2020
(51) Int. Cl.: A61K 45/06, A61K 9/28, A61K 9/48, A61K 36/287, A61K 36/45, A61K 36/63, A61K 36/67, A23L 33/105

(54) **MELANGE D'EXTRAITS DE PLANTES POUR SON UTILISATION DANS LA PREVENTION ET/OU LE TRAITEMENT DES MALADIES CHRONIQUES INFLAMMATOIRES DE L'INTESTIN**
PFLANZENEXTRAKTGEMISCH ZUR VERWENDUNG IN DER VORBEUGUNG UND/ODER BEHANDLUNG VON CHRONISCHEN ENTZÜNDLICHEN DARMERKRANKUNGEN
PLANT EXTRACT MIXTURE FOR USE IN THE PREVENTION AND/OR TREATMENT OF CHRONIC INFLAMMATORY BOWEL DISEASES

(30) Priorité: 11.06.2019 FR 1906165
(43) Date de publication de la demande: 20.04.2022
(73) Titulaire: Valbiotis, 17180 Perigny (FR)
(72) Inventeur: PELTIER, Sébastien, 17450 Fouras (FR); SIRVENT, Pascal, 63122 Ceyrat (FR); OTERO, Yolanda, 63000 Clermont-Ferrand (FR)
(74) Mandataire: Aquinov
(86) Numéro de dépôt international: PCT/EP2020/066164
(87) Numéro de publication internationale: WO 2020/249657

(56) Documents cités:
- WO-A1-2016/062958
- WO-A1-2016/156527
- WO-A1-2017/068069

## Description

### DOMAINE TECHNIQUE

La présente invention se rapporte à un mélange de plusieurs extraits de plantes pour son utilisation comme médicament ou produit de nutrition dans la prévention et/ou le traitement des maladies chroniques inflammatoires de l'intestin (MICI).

### ART ANTERIEUR

Les MICI, comme la maladie de Crohn ou la rectocolite hémorragique, sont des affections caractérisées par une inflammation de la paroi d'une partie du tube digestif liée à une hyperactivité du système immunitaire digestif. Les symptômes sont généralement caractérisés par des douleurs abdominales chroniques, de la diarrhée et une perte de poids. Ces maladies évoluent par poussées inflammatoires de durée et de fréquence extrêmement variables selon les patients. Ces poussées alternent avec des phases de rémission.

La prise en charge clinique permet la plupart du temps un contrôle durable de la maladie et une qualité de vie satisfaisante en dehors des poussées, mais il n'existe pas de traitement curatif. De plus, on sait que les traitements actuels ont des effets secondaires sur la densité osseuse, le gain de poids et même exacerbent la maladie intestinale (Asl Baakhtari S, McCombie A, Ten Bokkel Huinink S, Irving P, Siegel CA, Mulder R, et al. Observational Study of Perspectives of Inflammatory Bowel Disease Patients Concerning the Use of Corticosteroids. Dig Dis. 2018;36(1):33-9. Epub 2017/09/04. doi: 10.1159/000478772. PubMed PMID: 28866661 ; Waljee AK, Wiitala WL, Govani S, Stidham R, Saini S, Hou J, et al. Corticosteroid Use and Complications in a US Inflammatory Bowel Disease Cohort. PLoS One. 2016;11(6):e0158017. Epub 2016/06/24. doi: 10.1371/journal.pone.0158017. PubMed PMID: 27336296; PubMed Central PMCID: PMCPMC4918923***)**.*

La demande WO2016/156527 décrit le traitement de MICI en utilisant un hydrolysat de lactosérum et de caséine.

Le développement des MICI est lié à plusieurs facteurs (génétiques et environnementaux) mais son étiologie n'est pas complètement élucidée. Selon la pathologie considérée, l'étendue et la localisation de l'inflammation du tube digestif peuvent différer. Cette inflammation va conduire à une modification du temps de transit et à la perturbation de la fonction d'absorption intestinale de l'eau et des nutriments. En conséquence, la diarrhée représente l'un des symptômes les plus fréquents chez les patients atteints de MICI, qui peut notamment s'accompagner d'une présence de sang dans les selles lorsque des ulcérations sont présentes. De plus, la malabsorption intestinale est souvent à l'origine d'une perte de poids chez les patients atteints. Si les mécanismes physiopathologiques conduisant à ces perturbations physiologiques ne sont pas précisément connus, ils font l'objet de nombreuses recherches notamment sur des modèles animaux reconnus pour mimer cette symptomatologie. En exemple, des souris traitées avec du Sodium Dextran Sulfate (DSS) développent une inflammation intestinale accompagnée d'une élévation du score d'activité de la maladie (Disease Activity Score, DAI) prenant en compte l'évaluation de 3 symptômes majeurs retrouvés dans la pathologie humaine que sont la perte de poids, la consistance des fèces et le saignement, rendant ces modèles particulièrement intéressants pour l'identification de nouvelles pistes thérapeutiques *(*Randhawa PK, Singh K, Singh N, Jaggi AS. A review on chemical-induced inflammatory bowel disease models in rodents. Korean J Physiol Pharmacol. 2014 Aug;18(4):279-88. doi: 10.4196/kjpp.2014.18.4.279. Epub 2014 Aug 13. PubMed PMID: 25177159*).*

### EXPOSE DE L'INVENTION

L'objectif de l'invention est de proposer un produit capable d'agir sur ces symptômes et présentant une efficacité plus importante que les médicaments de référence dans la prévention et/ou le traitement des MICI.

Pour y répondre, l'invention vise l'utilisation d'une composition comprenant un mélange de plusieurs extraits de végétaux.

En particulier l'invention a pour objet une composition comprenant au moins :
- un extrait de Chrysanthellum, et
- un extrait d'artichaut, et
- un extrait de myrtillier, et
- un extrait d'au moins une matière première végétale comprenant de l'oleuropéine,
- et éventuellement de la pipérine synthétique et/ou un extrait d'au moins une matière première végétale comprenant de la pipérine
pour une utilisation comme médicament ou produit de nutrition dans la prévention et/ou le traitement des maladies inflammatoires chroniques de l'intestin chez l'être humain ou l'animal, notamment de la maladie de Crohn et/ou de la rectocolite hémorragique. Une telle composition est connue pour ses effets sur le métabolisme glucidique et/ou lipidique, mais de façon surprenante, selon l'invention, elle est capable de diminuer fortement l'index d'activité de la maladie de personnes ou d'animaux souffrant de MICI. Elle peut par conséquent constituer un médicament efficace pour la prévention et/ou le traitement des MICI. En effet, les extraits utilisés dans la composition selon l'invention sont notamment décrits dans WO2016/062958 et dans WO2017/068069, mais ces documents ne décrivent ni ne suggèrent un effet sur les MICI.

L'invention est à présent décrite en détails.

### DESCRIPTION DETAILLEE DE L'INVENTION

### Définitions

Par « analogues » (à un composé) au sens de la présente invention on entend tous composés ayant une structure chimique similaire à un autre composé, mais différent de celui-ci par un certain composant. Il peut différer de un ou plusieurs atomes, groupes fonctionnels, sous-structures, qui sont remplacés par d'autres atomes, groupes fonctionnels ou sous-structures. Par « extrait d'une plante « X » » au sens de l'invention, on entend un ensemble de molécules obtenu à partir d'une seule plante « X » par tout procédé adapté. On peut en particulier citer les extraits aqueux (obtenus à l'aide d'un solvant aqueux), alcooliques (obtenus à l'aide d'un solvant alcoolique) ou utilisant un solvant organique, ou à l'aide d'un corps gras naturel ou un mélange de corps gras naturels, notamment une huile végétale ou un mélange d'huiles végétales.

Par « mélange » au sens de l'invention on entend l'association de substances (extraits et/ou molécules) sous forme solide, liquide ou gazeuse qui peuvent interagir ou non chimiquement. Le mélange d'extraits selon l'invention est obtenu par tout procédé connu de l'Homme du métier. Il peut être obtenu par simple mélange des constituants.

Par « plante » ou « matière première végétale » au sens de l'invention, on entend la plante entière ou la partie de plante, incluant les cultures de cellules, n'ayant pas encore subi de traitement spécifique et destinée à entrer dans la fabrication d'une préparation de plante.

Par « produit de nutrition », on entend tous les produits ayant un effet nutritionnel et/ou physiologique, cela comprend notamment, les compléments alimentaires, les aliments, les produits diététiques, etc. Ces produits sont en particulier administrables par voie orale, gastrique ou veineuse.

Par « produit de santé », on entend tous les produits ayant un effet bénéfique pour la santé, en prévention ou en traitement, que cet effet soit physiologique ou pharmacologique, notamment les médicaments, les produits pharmaceutiques. Ces produits sont en particulier administrables par voie orale, gastrique, veineuse ou cutanée.

Par « solvant aqueux » au sens de l'invention, on entend tout solvant constitué totalement ou pour part d'eau. On peut citer ainsi l'eau elle-même, les solvants hydro-alcooliques en toute proportion ou encore les solvants constitués d'eau et d'un composé comme la glycérine ou le propylène glycol en toute proportion. Parmi les solvants alcooliques on peut citer notamment l'éthanol.

### Compositions

En particulier l'invention a pour objet une composition comprenant au moins :
- un extrait de Chrysanthellum, et
- un extrait d'artichaut, et
- un extrait de myrtillier, et
- un extrait d'au moins une matière première végétale comprenant de l'oleuropéine, et
- éventuellement de la pipérine synthétique et/ou un extrait d'au moins une matière première végétale comprenant de la pipérine
pour une utilisation comme médicament ou produit de nutrition dans la prévention et/ou le traitement des maladies inflammatoires chroniques de l'intestin chez l'être humain ou l'animal.

Dans la présente demande, le singulier ou le pluriel seront indifféremment utilisés pour désigner les compositions utiles selon l'invention.

La composition utile selon l'invention comprend donc un mélange de molécules comprenant plusieurs extraits de matières premières végétales, dont :
- au moins un extrait de Chrysanthellum, préférentiellement un extrait de *Chrysanthellum indicum,* et
- au moins un extrait d'artichaut, préférentiellement un extrait choisi parmi les extraits de *Cynara cardunculus* et les extraits de de *Cyanara scolymus,* et leurs mélanges, encore plus préférentiellement un extrait de *Cynara scolymus,* et
- au moins un extrait de myrtillier, préférentiellement un extrait de *Vaccinium,* notamment un extrait choisi parmi les extraits de *Vaccinium Myrtillus,* les extraits de *Vaccinium Angustifolium,* les extraits de *Vaccinium Corymbosum,* les extraits de *Vaccinium Ashei Reade,* et leurs mélanges, encore plus préférentiellement un extrait de *Vaccinium Myrtillus,* et
- un extrait d'au moins une matière première végétale comprenant de l'oleuropéine, préférentiellement un extrait choisi parmi les extraits d'Olivier (*Olea europeae*), les extraits de troène (*Ligustrum*), les extraits d'Arganier, et leurs mélanges, encore plus préférentiellement un extrait d'*Olea europeae,* et
- préférentiellement également de la pipérine synthétique et/ou un extrait d'au moins une matière première végétale comprenant de la pipérine, préférentiellement un extrait choisi parmi les extraits de *Piper,* les extraits de *Chalciporus piperatus,* et leurs mélanges.

Les extraits de plantes peuvent être obtenus par tout procédé adapté, par exemple par un procédé comprenant les étapes suivantes :
- extraction solide / liquide
- séparation / pressage
- filtration
- évaporation
- séchage
- éventuellement incorporation d'additifs
- homogénéisation
- conditionnement

L'extrait de *Chrysanthellum indicum* est de façon préférée un extrait de plante entière ou des parties aériennes. Il peut s'agir en particulier d'un extrait hydroalcoolique ou aqueux ou CO₂ subcritique ou H₂0 subcritique ou associé à un traitement thermique réalisé par chauffage classique ou sous fréquence micro-ondes ou sous ultrasons. Le ratio plante / extrait est préférentiellement compris entre 1/1 à 100/1, en particulier entre 1/1 et 25/1.

La composition selon l'invention, lorsqu'elle est destinée à l'Homme, comprend préférentiellement une quantité d'extrait de *Chrysanthellum indicum* permettant l'administration d'au moins 0,00001g, en particulier entre 0,00001g et 0,60g, d'extrait de *Chrysanthellum indicum* par kg de poids corporel de la personne à laquelle la composition est administrée et par jour.

De façon préférée, l'extrait de *Chrysanthellum indicum* comprend au moins une molécule choisie parmi l'apigenine-7-O-glucuronide, la chrysanthelline A, la chrysanthelline B, l'acide caféique, la lutéoline, la maritimétine, l'ériodictyol, l'isookanine, l'apigénine, la lutéoline-7-O-glucoside, la maritiméine, la maréine, l'ériodictyol-7-O-glucoside, la flavomaréine, l'apigénine-8-C-α-L-arabinoside-6-C-β-D-glucoside (shaftoside), l'apigénine-6,8-C-di-β-D-glucopyranoside (vicénine-2), ou leurs analogues. Préférentiellement l'extrait comprend au moins l'apigenine-7-O-glucuronide. On peut citer également par exemple les analogues de l'apigenine-7-O-glucuronide comme l'apigénine-7-apioglucoside, l'apigénine-8-C-glucoside (vitexine), l'apigénine-6-C-glucoside (isovitexine), l'apigénine-7-O-néohespéridoside, l'apigénine-7-glucoside, l'apigénine-7-apioglucoside.

L'extrait de *Cynara cardunculus* ou de *Cyanara scolymus,* est de façon préférée un extrait de feuilles ou de racines. Il peut s'agir en particulier d'un extrait hydroalcoolique ou aqueux ou CO₂ subcritique ou H₂0 subcritique ou associé à un traitement thermique réalisé par chauffage classique ou sous fréquence micro-ondes ou sous ultrasons. Le ratio plante / extrait est préférentiellement compris entre 1/1 à 100/1, en particulier entre 1/1 et 30/1.

La composition selon l'invention, lorsqu'elle est destinée à l'Homme, comprend préférentiellement une quantité d'extrait de *Cynara cardunculus* ou de *Cyanara scolymus* permettant l'administration d'au moins 0,00001g, en particulier entre 0,00001g et 0,60g, d'extrait de *Cynara cardunculus* ou de *Cyanara scolymus* par kg de poids corporel de la personne à laquelle la composition est administrée et par jour.

De façon préférée, l'extrait de *Cynara cardunculus* ou de *Cyanara scolymus* comprend au moins une molécule choisie parmi un acide dicaféoylquinique, un acide sulfo-monocaféoylquinique, la lutéoline, la lutéoline-7-0-glucoside, la lutéoline-7-O-glucuronide, l'apigenine-7-O-glucoside, la cynaropicrine, ou leurs analogues. Préférentiellement, l'extrait comprend au moins un acide dicaféoylquinique.

L'extrait de *Vaccinium* est préférentiellement un extrait de fruits ou de feuilles. Il peut s'agir en particulier d'un extrait hydroalcoolique ou aqueux ou CO₂ subcritique ou H₂0 subcritique ou associé à un traitement thermique réalisé par chauffage classique ou sous fréquence micro-ondes ou sous ultrasons. Le ratio plante / extrait est préférentiellement compris entre 1/1 à 200/1, en particulier entre 1/1 et 60/1.

La composition selon l'invention, lorsqu'elle est destinée à l'Homme, comprend préférentiellement une quantité d'extrait de *Vaccinium* permettant l'administration d'au moins 0,00001g, en particulier entre 0,00001g et 0,60g, d'extrait de *Vaccinium* par kg de poids corporel de la personne à laquelle la composition est administrée et par jour.

De façon préférée, l'extrait de *Vaccinium* comprend au moins une molécule choisie parmi un acide monocaféoylquinique, la delphinidine-3-galactoside, la delphinidine-3-glucoside, la cyanidine-3-galactoside, la delphinidine-3-arabinoside, la cyanidine-3-glucoside, la petunidine-3-galactoside, la cyanidine-3-arabinoside, la petunidine-3-glucoside, la péonidine-3-galactoside, la petunidine-3-arabinoside, la péonidine-3-glucoside, la malvidine-3-galactoside, la malvidine-3-glucoside, la malvidine-3-arabinoside, ou leurs analogues. Préférentiellement, l'extrait comprend au moins un acide monocaféoylquinique.

La composition selon l'invention, lorsqu'elle est destinée à l'Homme, comprend préférentiellement une quantité de pipérine permettant l'administration d'au moins 0,001mg, en particulier entre 0,001mg et 166 mg, de pipérine par kg de poids corporel de la personne à laquelle la composition est administrée et par jour. Si la pipérine est contenue dans un extrait de *Piper,* le mélange de la composition selon l'invention comprend ledit extrait. L'extrait de *Piper* est préférentiellement un extrait de *Piper nigrum,* de *Piper aduncum* et/ou de *Piper longum.*

L'extrait de *Piper* est de façon préférée un extrait de fruits ou de feuilles. Il peut s'agir en particulier d'un extrait hydroalcoolique ou aqueux ou CO₂ subcritique ou H₂0 subcritique ou associé à un traitement thermique réalisé par chauffage classique ou sous fréquence micro-ondes ou sous ultrasons. Le ratio plante / extrait est préférentiellement compris entre 1/1 et 10000/1, en particulier entre 1/1 et 200/1.

L'extrait comprend préférentiellement au moins 1% de pipérine en poids par rapport au poids total de l'extrait.

Si l'oleuropéine est contenu dans un extrait d'*Olea europaea,* ledit extrait est préférentiellement un extrait de feuilles ou de fruits. Il peut s'agir en particulier d'un extrait hydroalcoolique ou aqueux ou CO₂ subcritique ou H₂0 subcritique ou associé à un traitement thermique réalisé par chauffage classique ou sous fréquence micro-ondes ou sous ultrasons. Le ratio plante / extrait est préférentiellement compris entre 1/1 à 200/1, en particulier entre 1/1 et 60/1.

La composition selon l'invention, lorsqu'elle est destinée à l'Homme, comprend préférentiellement une quantité d'extrait d'*Olea europaea* permettant l'administration d'au moins 0,00001g, en particulier entre 0,00001g et 0,60g, d'extrait d'*Olea europaea* par kg de poids corporel de la personne à laquelle la composition est administrée et par jour.

De façon préférée, l'extrait d'*Olea europaea* comprend au moins une molécule en l'hydroxytyrosol ou un analogue, en plus de l'oleuropéine.

De façon préférée, la composition selon l'invention comprend au moins les molécules suivantes (ces molécules étant comprises dans le ou les extraits constituant la composition selon l'invention) :
- au moins une molécule choisie parmi l'apigenine-7-O-glucuronide, la chrysanthelline A, la chrysanthelline B, l'acide caféique, la lutéoline, la maritimétine, l'ériodictyol, l'isookanine, l'apigénine, la lutéoline-7-O-glucoside, la maritiméine, la maréine, l'ériodictyol-7-O-glucoside, la flavomaréine, l'apigénine-8-C-α-L-arabinoside-6-C-β-D-glucoside (shaftoside), l'apigénine-6,8-C-di-β-D-glucopyranoside (vicénine-2), ou leurs analogues, préférentiellement l'apigenine-7-O-glucuronide, et

- au moins une molécule choisie parmi un acide dicaféoylquinique, un acide sulfo-monocaféoylquinique, la lutéoline, la lutéoline-7-0-glucoside, la lutéoline-7-O-glucuronide, l'apigenine-7-O-glucoside, la cynaropicrine, ou leurs analogues, préférentiellement un acide dicaféoylquinique, et
- au moins une molécule choisie parmi un acide monocaféoylquinique, la delphinidine-3-galactoside, la delphinidine-3-glucoside, la cyanidine-3-galactoside, la delphinidine-3-arabinoside, la cyanidine-3-glucoside, la petunidine-3-galactoside, la cyanidine-3-arabinoside, la petunidine-3-glucoside, la péonidine-3-galactoside, la petunidine-3-arabinoside, la péonidine-3-glucoside, la malvidine-3-galactoside, la malvidine-3-glucoside, la malvidine-3-arabinoside, ou leurs analogues, préférentiellement au moins un acide monocaféoylquinique, et
- de l'oleuropéine, et
- éventuellement préférentiellement également du verbascoside et/ou de la luteolin-7-o-glucuronide, et
- éventuellement de la pipérine.

Selon une variante particulièrement adaptée, le mélange d'extraits comprend au moins un acide dicaféoylquinique, du verbascoside, l'apigenine-7-O-glucuronide, de la luteolin-7-o-glucuronide, un acide monocaféoylquinique, l'oleuropéine et éventuellement de la pipérine.

A titre alternatif et équivalent une ou plusieurs ou la totalité des molécules de l'extrait unique peuvent être remplacées par des molécules de synthèse ou naturelles provenant d'autres plantes.

Les compositions selon l'invention sous leurs différentes variantes, peuvent être exclusivement constituées des éléments décrits (extraits de plantes), qui constituent ensemble un principe actif, ou bien peuvent comprendre également au moins un élément supplémentaire (produits, molécules, extraits, principes actifs, excipients, etc) ajouté en plus des extraits de plantes, ledit élément supplémentaire pouvant être choisi parmi :
- les vitamines suivantes : B1, B2, B3, B5, B6, B8, B9, B12 C, A, D, E, K1 et K2 ;
- les composés suivants : acide obeticholique, acide corosolique, acides gras polyinsaturés de la famille des oméga 6 et/ou oméga 3, acide orotique, acide pangamique, acide para-amino-benzoïque, amygdaline, béta-glucanes, carnitine, diméthylglycine, imeglimine, isoflavones, L-arginine, oxytocine, pectine, pyridoxamine, resvératrol, viniférine, L-citrulline ;
- les oligo-éléments et minéraux suivants : arsenic, bore, calcium, cuivre, fer, fluor, iode, lithium, manganèse, magnésium, molybdène, nickel, phosphore, sélénium, vanadium, zinc ;
- les microconstituants à caractère non indispensable suivants : acide linolénique conjugué, acide lipoïque, caroténoïdes, carnitine, choline, coenzyme Q10, phytostérols, polyphénols de la famille des tannins et des lignanes, taurine ;
- des pré-biotiques par exemple des fructo-oligosaccharides, des inulines, des galacto-oligosaccharides, des pectines, des bêta-glucanes, et des xylooligosacharides ;
- des probiotiques par exemple des Lactobacillus, Bifidobacteria, Akkermansia, Clostridium, Escherichia, Faecalibacteria, Bacteroides, Eubacteria ;
- les anti-inflammatoires, les corticoïdes (par exemple budésonide, betamethasone, prednisolone, et des autres dérivés de la corticosterone), les dérivés aminosalicylés ;
- des anticorps monoclonaux, par exemple infliximab, adalimumab, védolizumab; des dérivés aminosalicylés, par exemple mésalazine ;
- des levures, par exemple de la levure de riz rouge (*Monascus purpureus*) ;
- des champignons, par exemple le maïtaké ;
- les produits dérivés d'insectes compatibles avec le secteur alimentaire et pharmaceutique ;
- la marijuana et le haschisch ;
- des agents d'enrobage, par exemple l'hypromellose, la cellulose microcristalline, l'acide stéarique, du talc, du sucre, de la gomme laque, du povidone, de la cire d'abeille ;
- des arômes, par exemple d'arôme naturel de myrtille ou d'arôme naturel de fraise ;
- des acidifiants comme l'acide malique ;
- des antiagglomérants, par exemple le dioxyde de silicium ou le stéarate de magnésium ;
- des épaississants comme la gomme de xanthane, la silice colloïdale, les mono et diglycérides d'acides gras ;
- des stabilisants comme le phosphate de calcium ;
- des émulsifiants comme la lécithine de soja ;
- des agents de charge comme l'amidon de maïs ;
- des excipients, par exemple de la cellulose microcristalline, du stéarate de magnésium ou du phosphate dicalcique.

Les compositions selon l'invention peuvent également comprendre un ou plusieurs extraits d'au moins une des matières premières végétales suivantes et/ou une ou plusieurs molécules contenues dans au moins une des matières végétales suivantes : *Abelmoschus esculentus, Abies Alba, Abies balsamea, Abies sibirica, Acacia nilotica, Acacia senegal, Achillea millefollium, Achyranthes bidentata, Acmella oleracea, Actaea racemosa, Actinidia chinensis, Actinidia deliciosa, Adansonia digitata, Adiantum capillus-veneris, Aesculus hippocastanum, Afromomum melegueta, Agathosma betulina, Agathosma crenulata, Agathosma serratifolia, Agrimonia eupatoria, Ajuga reptans, Albizia julibrissin, Alchemilla vulgaris, Alliara petiolata, Allium ampeloprasum, Allium cepa, Allium sativum, Allium schoenoprasum, Allium ursinum, Alnus glutinosa, Aloe ferox, Aloe vera, Aloysia citriodora, Alpinia galanga, Alpinia hainanensis, Alpinia officinarum, Alpinia oxyphylla, Althaea officinalis, Ammi visnaga, Amorphophallus konjac, Ananas comosus, Andographis paniculata, Anemarrhena asphodeloides, Anethum graveolens, Angelica archangelica, Angelica dahurica, Angelica pubescens, Angelica sinensis, Antennaria diocia, Anthriscus cerefolium, Anthyllis vulneraria, Aphanizomenon flos-aquae Ralfs, Apium graveolens, Arachis hypogaea, Aralia elata, Arctium lappa, Arctium minus, Argania spinosa, Armorica rustanica, Artemisia dracunculus, Artemesia vulgaris, Ascophyllum nodosum, Aspalathus linearis, Asparagus officinalis, Astragalus membranaceus, Atractylodes lancea, Atractylodes macrocephala, Auracaria columnaris, Avena* staiva, Ayahuasca, *Baccharis genistelloides, Bacopa monnierri, Ballota nigra, Bambusa bambos, Bellis perennis, Berberis vulgaris, Beta vulgaris, Betula alleghaniensis, Betula pendula, Betula pubescens, Bixa orellana, Borago officnalis, Boswellia serrata, Brassica napus, Brassica nigra, Brassica oleracea, Brassica rapa, Bupleurum chinense, Calendula officinalis, Calluna vulgaris, Camellia sinsensis, Capsella bursa-pastoris, Capsicum annuum, Carex arenaria, Carica papaya, Carlina acaulis, Carphephorus odoratissmus, Carpinus betulus, Carthamus tinctorius, Carum carvi, Cassia fistula, Castanea sativa, Centaurea centaurium, Centaurea cyanus, Centaurium erythraea, Centella asiatica, Cerasus vulgaris, Ceratonia silliqua, Chaenomelum nobile, Chlorella vulgaris, Chondrus crispus, Chrysanthellum indicum, Cichorium intybus, Cinchona officinalis, cinchona pubescens, Cinnamomum camphora, Cinnamomum cassia, Cinnamomum verum, Cistanche salsa, Cistus incanus, Citrus aurantium, Citrus limon, Citrus maxima, Citrus medica, Citrus myrtifolia, Citrus reticulata blanco, Citrus sinsensis, Citrus paradisi, Clinopodium vulgare, Cnicus benedictus, Cochlearia officinalis, Cocos nucifera, Codonopsis pilosula, Coffea canephora, Coix lacryma-jobi var. mayyuen Stapf, Cola acuminata, Cola ballayi cornu, Cola nitida, Combretum micranthum, Commiphora mukul, Conyza canadensis, Coriandrum sativum, Cornus officinalis, Corylus avellana, Corymbia citriodora, Crataegus laevigata, Craetegus monogyna, Crithmum maritimum, Crocus sativus, Cucumis melo, Cucurbita pepo, Cuminum cyminum, Cupressus sempervirens, Cuscuta chinensis, Cyamopsis tetragonoloba, Cyathula officinalis, Cyclanthera pedata, Cydonia oblonga, Cymbopogon martini, Cymbopogon nardus, Cymbopogon winterianus, Cynara cardunculus, Cyperus rotundus, Daucus carota, Dendranthema grandiflorum, Desmodium adscendens, Dimocarpus longan, Dioscorea oppostifolia, Dioscorea villosa, Diospyros kaki Thunb., Dunaliella saliena, Echinacea augustifolia, Echinacea pallida, Echinacea purpurea, Elaegnus rhamnoides, Alettaria cardamomum, Eleutherococcus senticosus, Elymus repens, Epiobium augustifolium, Epilobium parviflorum, Equisetum arvense, Erica cinerea, Erica tetralix, Eriobotrya japonica, Eriodictyon californicum, Erodium cicutarium, Eryngium campestre, Eschscholzia californica, Eucalyptus dives Schauer, Eucalyptus globulus, Eucalyptus radiata, Eucalyptus smithii F. Muell, Eucommia ulmoides, Eugenia uniflora, Eugenia jambolana, Euphrasia stricta D. Wolff, Euterpe oleracea, Fagopyrum esculentum Moench, Follopia japonica, Ferula assa-foetida, Ficus carica, Filipendula ulmaria, Foeniculum vulgare Mill., Forsythia suspensa, Fragaria dodonei Ard., Frangula purshiana Cooper, Fraxinus excelsior, Fraxinus ortus, Fucus serratus, Fucus vesiculosus, Fumaria officinalis, Galeopsis segetum Neck., Galium odotarum, Galium verum, Gardenia jasminoides J. Ellis, Gastrodia elata Blume, Gelidium corneum J.V. Lamouroux, Gentiana lutea, Geranium robertianum, Geum urbanum, Ginkgo biloba, Glycine max, Glycyrrhiza glabra, Glycyrrhiza uralensis, Gracilaria gracilis, Grindelia camporum Greene, Grindelia robusta Nutt., Grindelia squarrosa Dunal, Gymnema sylvestris, Haematococcus pluvialis, Hamamemis virginiana, Harpagophytum procumbens, Harpagophytum zeyheri Decne., Hedeoma pluegioides Pers., Helianthus annuus, Helienthus tuberosus, Helichrysum arenarium, Helichrysum stoechas, Herniara glabra, Hibiscus sabdariffa, Hieracium pilosella, Himanthalia elongata, Hordeum vulgare, Houttuynia cordata Thunb., Huperzia serrata, Hyssopus officinalis, Ilex paraguariensis A. St.-Hill, Illicum verum, Impatients balsamina, Inula britannica, Inula helenium, Jasminum grandiflorum, Jasmium officinale, Juniperus commuais, Justicia adhatoda, Kavalama urens, Krameria lappacea, Lagerstroemia speciosa, Laminaria digitata, Laminaria hyperborea, Lamium album, Larix decidua, Larix occidentalis, Laurus nobilis, Lavandula augustofolia, Lavandula latifolia, Ledum palustre, Leonurus cardiaca, Lepidium meyenii Walp., Lepidium sativum, Lespedeza capitata, Levisticum officinale, Lindera aggregata, Linus usitatissimum, Liquidambar styraciflua, Lotus corniculatus, Lycium chinense, Lycium barbarum, Lycopersicon esculentum, Lycopodium clavatum, Lycopus europaeus, Lythrum salicaria, Macadamia ternifolia F. muell, Macrocystis pyrifera, Magnolia officinalis, Malpighia glabra, Malus pumila, Malus domestica, Malus sylvestris, Malva sylvestris, Mangifera indica, Maranta arundinacea, Marrubium vulgare, Marsdenia cundurango, Marsdenia sylvestris, Mastocarpus stellatus, Matricaria chamomilla, Medicago sativa, Melaleuca alternifolia, Melaleuca cajuputi Powell, Melaleuca leucadendra, Melaleuca quinquenrvia, Melaleuca viridiflora, Melilotus altissimus Thuill., Melilotus officinalis, Mentha arvensis, Mentha x piperita, Menyanthes trifoliata, Mesembryanthemum crystallinum, Monarda didyma, Morinda citrifolia, Morinda officinalis, Morus alba, Morus nigra, Murraya koenigii, Musa x paradisiaca, Myrciaria dubia, Myristica flagrans Houtt., Myroxylon balsamum, Myrtus communis, Nardostachys jatamansi, Nasturtium officinale R. Br., Nelumbo nucifera Gaertn., Nepeta cataria, Nepeta tenuifolia Benth., Nigella sativa, Ocimum basilicum, Oenothera biennis, Ononis spinosa, ophiopogon japonicus, Opuntia ficus-indica, Origanum compactum Benth., Origanum majorana, Origanum vulgare, Orthosiphon aristatus, Oryza sativa, Paeonia lactiflora, Paeonia x suffruticosa Andrews, Palmaria palmata, Panax ginseng, Panax quinquefolius, Panicum miliacium, Papaver rhoeas, Parietaria officinalis, Passiflora edulis Sims, Pastinaca sativa, Paullinia cupana Kunth, Pelargonium graveolens, Perilla frutescens, Persea americana, Persicaria bistorta, Persicaria maculosa Gray, Petroselinum crispum, Peucadanum ostruthium, Peumus boldus Molina, Phaseolus vulgaris, Phellodendron amurense, Photinia melancarpa, Phyllanthus emblica, Physalis alkekengi, Phymatolithon calcareum, Picea abies, Pimenta dioca, Pimenta racemosa, Pimpinella anisum, Pimpinella major, Pimpinella saxfraga, Pinus mugo Turra, Pinus pinasterAiton, Pinus sylvestris, Pistacia lentiscus, Plantago arenaria, Plantago lanceolata, Plantago major, Plantago ovata, Platycodon grandiflorus, Plectranthus barbatus Andrews, Pogostemom cablin, Polygala senega, Polygala sibirica, Polygala tenuifolia Willd., Polygonum aviculare, Populus nigra, Populus tremula, Populus tremuloides, Porphyre umbilicalis, Portulaca oleracea, Potentilla erecta, Primula veris, Prunella vulgaris, Prunus africana, Prunus armeniaca, Ribes nigrum, Ribes uva-crispa, Rosa canina, Rosa gallica, Rosa moschata, Rosa rubiginosa, Rosmarinus officinalis, Rubus caesius, Rubus fruticosus, Rubus idaeus, Rumex actetosa, Rumex acetosella, Rumex crispus, Rumex patienta, Ruscus aculeatus, Sachharina japonica, Saccharina latissima, Salix alba, Salix fragilis, Salix pentandra, Salix purpurea, Salvia officinalis L., Salvia officinalis subsp. lavandulifolia Gams, Salvia sclarea, Sambucus nigra, Sanguisorba officinalis, Sanicula elata Buch.-Ham. Ex D. Don, Santalum album, Santolina chamaecyparissus, Saposhnikovia divaricata, Sargassum fusiforme, Satureja hortensis, Satureja montana, Saussurea costus, Scrophularia ningpoensis Helmsl., Scutellaria baicalensis Georgi, Secale cereale, Sedum acre, Sedum roseum, Senna alexandrina Mill., Senna obustifolia, Smilax cordifolia Humb.* & *Bonpl., Smilax glabra Roxb., Smilax officinalis Kunth, Smilax purhampuy Ruiz, Smilax purhampuy Ruiz, Smilax regelli Killip et C.V. Morton, Smilax vanillidora Apt, Solanum melongena, Solanum tuberosum, Solidago virgaurea, Sorbus aucuparia, Spatholobus suberctus Dunn., Spinacia oleracea, Spirulina major Kützing, Spirulina maxima Geitler, Spirulina platensis Geitler, Stavhys officinalis, Stemmacantha carthamoides Dittrich, Stypholobium japonicum, Syzgium aromaticum, Tagetes erecta, Tamarindus indica, Tanacetum parthemium, Terminalia chebula Retz., Theobroma cacao, Thymus saturejoides Coss., Thymus serpyllum, Thymus vulgaris, Thymus zygis, Tilia cordai-a Mill., Tilia platyphyllos Scop., Tilia tomentosa Moench, Tilia euopaea, Tribulus terres tris, Trichosanthes kirilowii Maxim., Trifolium arvense, Trifolium campestre Schreb., Trifolium pratense, Trifolium repens, Trigonella caerulea, Trigonella foenum-graecum, Tricitum aestivum, Tricitum durum Desf., Tricitum spelta L., Tricitum turgidum, Tropaeolum majus, Turnera diffusa Willd., Ulmus glabra Huds., Ulmus glabra Huds., Ulmus pumila, Ulmus rubra Muhl., Ulva lactuca, Uncaria gambir Roxb., Uncaria rhynchophylla Miq., Uncaria tomentosa DC., Undaria pinnatifida Suringar, Urtica dioca, Urtica urens, Vaccinium macrocarpon, Vaccinium oxycoccos, Vaccinium vitis-idae, Valeriana jatamansi Jones, Valeriana officinalis, Vanilla planifolia Jacks, Verbascum densiflorum Bertol., Verbascum thapsus, Verbena officinalis, Veronica officinalis, Viburnum opulus, Vigna angularis Ohwi & H. Ohashi, Vinca major, Vinca minor, Viola palustris, Viola tricolor, Vitex agnus-castus, Vitex trifolia, Vitis vinifera, Zea mays, Zingiber officinale Roscoe, Ziziphus jujuba Mill.*

### Utilisations

Les compositions selon l'invention sont destinées à être administrées à l'Homme ou l'animal par tout moyen d'administration, préférentiellement par voie orale. Elles peuvent se présenter sous forme de poudre, de gel, d'émulsion, ou sous forme liquide, et en particulier sous forme de comprimés, capsules, gélules, sticks, sachets, ampoules, compte-gouttes ou sous forme injectable.

Les compositions selon l'invention peuvent être utilisées comme produits de nutrition ou produits de santé en particulier comme médicament.

Lorsqu'elles sont utilisées chez une personne ou un animal atteint de MICI, elles conduisent à une réduction du score d'activité de la maladie prenant en compte 3 composantes majeures de la symptomatologie observée chez l'Homme : la perte de poids, le saignement et la texture des fèces.

Cet effet permet ainsi à la composition selon l'invention d'être utilisée dans la prévention et/ou le traitement des maladies inflammatoires chroniques de l'intestin chez l'être humain ou l'animal, en particulier dans la prévention et/ou le traitement des douleurs abdominales chroniques et/ou de la diarrhée et/ou de la perte de poids des maladies inflammatoires chroniques de l'intestin. La composition selon l'invention peut notamment être utilisée dans la prévention et/ou le traitement de la maladie de Crohn et/ou de la rectocolite hémorragique.

L'invention est à présent illustrée par des exemples d'extraits et de compositions, ainsi que par des résultats d'essais démontrant l'efficacité des compositions selon l'invention, ces exemples et essais n'étant pas limitatifs.

### EXEMPLES

### Exemple 1 : exemple d'extrait sec de Chrysanthellum indicum pour intégrer dans une composition selon l'invention

On soumet les parties aériennes de la plante, fraîche ou sèche, à un broyage mécanique jusqu'à obtention d'une poudre grossière. Cette poudre est ensuite soumise à une étape de macération pendant 10 à 24 heures à température ambiante dans un mélange 70/10 eau / éthanol, puis on fait subir à l'ensemble obtenu une lixiviation en continu à 50°C dans un percolateur avec un mélange 70/10 eau / éthanol, le rapport plante / extrait étant de 3/1. L'extrait obtenu est ensuite soumis à des lavages liquide / liquide à l'aide d'un solvant organique non polaire comme le di- ou le tri-chlorométhane. Après concentration par évaporation à basse pression à 35°C, on obtient un liquide qui, lyophilisé pendant 24 heures, donne une poudre beige soluble dans un mélange eau / alcool. Cette poudre (extrait sec) peut être utilisée directement ou bien mélangée dans un solvant approprié avant utilisation.

### Exemple 2 : exemple d'extrait sec de Vaccinium myrtillus pour intégrer dans une composition selon l'invention

La myrtille sous forme de poudre obtenue à partir de fruits de *Vaccinium myrtillus,* est soumise à une étape de macération pendant 10 à 24 heures à température ambiante dans un mélange 30/50 eau / éthanol, puis on fait subir à l'ensemble obtenu une lixiviation en continu à 50°C dans un percolateur avec un mélange 30/50 eau / éthanol, le rapport plante / extrait étant de 10/1. L'extrait obtenu est ensuite soumis à des lavages liquide / liquide à l'aide d'un solvant organique non polaire comme le di- ou le tri-chloromëthane. Après concentration par évaporation à basse pression à 35°C, on obtient un liquide qui, lyophilisé pendant 24 heures, donne une poudre de couleur violette soluble dans un mélange eau / alcool.

### Exemple 3 : exemple d'extrait sec de Cynara scolymus pour intégrer dans une composition selon l'invention

L'artichaut sous forme de poudre obtenue à partir de feuilles de *Cynara scolymus* est soumis à une étape de macération pendant 10 à 24 heures à température ambiante dans de l'eau, puis on fait subir à l'ensemble obtenu une lixiviation en continu à 50°C dans un percolateur avec de l'eau, le rapport plante / extrait étant de 2/1. L'extrait obtenu, est ensuite soumis à des lavages liquide / liquide à l'aide d'un solvant organique non polaire comme le di- ou le tri-chloromëthane. Après concentration par évaporation à basse pression à 35°C, on obtient un liquide qui, lyophilisé pendant 24 heures, donne une poudre beige soluble dans l'eau.

### Exemple 4 : exemple d'extrait sec de Piper nigrum pour intégrer dans une composition selon l'invention

On soumet la partie fruit de la plante, fraîche ou sèche, à un broyage mécanique jusqu'à obtention d'une poudre grossière. Les molécules d'intérêt, présentes dans cette poudre grossière, sont ensuite extraites par un mélange eau/éthanol. L'extrait obtenu est ensuite soumis à des étapes d'évaporation du solvant, concentration, recristallisation, filtration et lavages. Si cela est nécessaire ces étapes sont répétées plusieurs fois pour optimiser le rendement d'extraction. Après une dernière étape de recristallisation et filtration en milieu aqueux, le précipité obtenu est séché et récupéré.

### Exemple 5 : exemple d'extrait sec d'Olea europaea pour intégrer dans une composition selon l'invention

Les feuilles d'olivier entières et séchées à l'air sont broyées à -80°C à l'aide d'un broyeur à couteaux pour obtenir une poudre fine et homogène. La poudre obtenue est ensuite soumise à une étape de macération pendant 10 à 24 heures dans un mélange 70/30 eau / éthanol. L'étape est conduite en système fermé avec bullage d'azote à température ambiante, ou sous une puissance de micro-ondes de 800 watts ou sous une fréquence d'ultrasons de 20 kHz pendant 2 × 3 min. On fait ensuite subir à l'ensemble obtenu une lixiviation en continu à 50°C dans un percolateur avec un mélange 70/30 eau / éthanol, le rapport plante / extrait étant de 10/1. L'extrait obtenu est ensuite soumis à des lavages liquide / liquide à l'aide d'un solvant organique non polaire comme le di- ou le tri-chloromëthane. Après concentration par évaporation à basse pression à 35°C, on obtient un liquide qui, lyophilisé pendant 24 heures, donne une poudre de couleur verte dans un mélange eau / alcool.

### Exemple 6 : exemple de composition selon l'invention sous forme de gélules

La composition de l'exemple 6 se présente sous forme de gélules administrables par voie orale. Elle comprend, exprimés en pourcentage en poids, rapporté au poids total de la composition, 37,0% d'extrait sec de l'exemple 1, 37,0% d'extrait sec de l'exemple 1, 3,7% d'extrait sec de l'exemple 3, 0,004% d'extrait sec de l'exemple 4, et 22,2% d'extrait sec de l'exemple 5.

La composition pour 3 gélules est indiquée dans le **Tableau 1** suivant.

**[Tableau 1]**

| **Li**s**te des ingrédients** | **Pour 3 gélules** |
|---|---|
| Extrait sec de parties aériennes de *Chrysanthellum indicum* | 200 mg |
| Extrait sec de feuilles de *Cynara scolymus* | 200 mg |
| Extrait sec de fruits de *Vaccinium myrtillus* | 20 mg |
| Extrait sec de fruits de *Piper nigrum* | 0,02 mg |
| Extrait sec de feuilles d'*Olea europaea* | 120 mg |

### Exemple 7 : exemple de composition selon l'invention sous forme de comprimés

La composition de l'exemple 7 se présente sous forme de comprimés administrables par voie orale. Elle comprend, exprimés en pourcentage en poids, rapporté au poids total de la composition, 22,0% d'extrait sec de parties aériennes de *Chrysanthellum indicum,* 22,0% d'extrait sec de feuilles de *Cynara scolymus,* 2,2% d'extrait sec de fruits de *Vaccinium myrtillus*, 13,2% d'extrait sec de feuilles d'*Olea europaea,* et 0,2% d'extrait sec de fruits de *Piper nigrum.* La composition comprend également en plus du mélange du zinc, les vitamines B9, PP, B5, H, B12, D, B6, B2, B2 et du chrome. Elle comprend également des excipients en particulier du phosphate dicalcique, de la cellulose microcristalline et du
stéarate de magnésium.

La composition pour 1 comprimé est indiquée dans le **Tableau 2** suivant.

**[Tableau 2]**

| **Liste des ingrédients** | **En mg pour 1 comprimé** | **Valeur Nutritionnelle de Référence pour 1 comprimé** |
|---|---|---|
| Extrait sec de parties aériennes de *Chrysanthellum indicum* | 220,0 | - |
| Extrait sec de feuilles de *Cynara scolymus* | 220,0 | - |
| Extrait sec de fruits de *Vaccinium myrtillus* | 22,0 | - |
| Extrait sec de feuilles d'*Olea europaea* | 132,0 | - |
| Extrait sec de fruits de *Piper nigrum* | 2,0 | - |
| Phosphate dicalcique | 198,0 | - |
| Cellulose microcristalline | 153,44 | - |
| Stéarate de magnésium | 20,0 | - |
| Bisglycinate de zinc | 12,99 | 33% |
| Vitamine B9 - acide folique | 7,30 | 33% |
| Vitamine PP - nicotinamide | 5,30 | 33,1% |
| Vitamine B5 - calcium pantothénate | 2,24 | 33,3 |
| Vitamine H - biotine | 1,66 | 33,2% |
| Vitamine B12 | 0,83 | 33,2% |
| Vitamine D3 | 0,64 | 32% |
| Vitamine B6 - pyridoxine chlorhydrate | 0,56 | 32,9% |
| Vitamine B1 - thiamine chlorhydrate | 0,48 | 32,7% |
| Vitamine B2 - riboflavine | 0,45 | 32,1% |
| Picolinate de chrome | 0,11 | 32,5% |

### Exemple 8 : exemple de composition selon l'invention sous forme de poudre à reconstituer dans l'eau, conditionnée sous forme de sticks

La composition de l'exemple 8 se présente sous forme de poudre à reconstituer dans de l'eau, conditionnée sous forme de sticks administrables par voie orale. Elle comprend, exprimés en pourcentage en poids, rapporté au poids total de la composition, 37,0% d'extrait sec de parties aériennes de *Chrysanthellum indicum* (exemple 1), 37,0% d'extrait sec de feuilles de *Cynara scolymus* (exemple 2), 3,7% d'extrait sec de fruits de *Vaccinium myrtillus* (exemple 3), 0,2% d'extrait sec de fruits de *Piper nigrum* (exemple 4), et 22,2% d'extrait sec de feuilles *d'Olea europaea* (exemple 5). La composition comprend également de la vitamine B12 et du chrome. A titre d'arôme, elle comprend un arôme naturel de fraise. A titre d'antiagglomérant, elle comprend du dioxyde de silicium. Cette composition ne comprend pas d'antiagglomérant et d'épaississant.

La composition d'un tel produit est indiquée dans le **Tableau 3** suivant.

**[Tableau 3]**

| Liste des ingrédients | Pour 3 sticks | Valeur Nutritionnelle de Référence pour 3 sticks |
|---|---|---|
| Extrait sec de fruits de *Vaccinium myrtillus* | 120 mg | - |
| Extrait sec de parties aériennes de *Chrysanthellum indicum* | 1200 mg | - |
| Extrait sec de feuilles de *Cynara scolymus* | 1200 mg | - |
| Extrait sec de fruits de *Piper nigrum* | 6 mg | |
| Extrait sec de feuilles d'*Olea europaea* | 720 mg | - |
| Vitamine B3 (hexanicotinate d'inositol) | 2,5 µg | 100% |
| Chrome (picolinate de chrome) | 20 µg | 50% |

### EVALUATION IN VIVO DE l'EFFICACITE DE LA COMPOSITION

Des expérimentations *in vivo* chez l'animal ont été conduites de manière à évaluer l'efficacité de la composition selon l'invention sur la prévention et le développement des MICI. Le modèle choisi était la souris nourrie avec un régime riche en lipides et soumise à l'ajout de Dextran Sulfate Sodium (DSS, agent inflammatoire chimique) dans l'eau de boisson pendant 7 jours. Ce modèle est reconnu pour induire une dysbiose intestinale et une inflammation similaire à celle retrouvée chez l'Homme dans le contexte des MICI (Eichele DD, Kharbanda KK, World J Gastroenterol. 2017 Sep 7;23(33):6016-6029. doi: 10.3748/wjg.v23.i33.6016 *;* Lee J, Lee H, Kim T, Kim M, Park Y, Kim J, Park K et al. Plos One 2017. 12(11):e0187515. doi: 10.1371/journal.pone.0187515).

Des souris males C57BI/6 âgées de 8 semaines ont été nourries avec un régime riche en graisses pendant 5 semaines. Pendant la dernière semaine, 2% de DSS a été ajouté dans l'eau de boisson. Un groupe de souris était également supplémenté avec l'incorporation dans ce régime riche en lipide d'une composition comprenant un mélange des extraits des exemples 1 à 5, dans un ratio *Chrysanthellum indicum* / *Cynara scolymus* / *Vaccinium myrtillus* / *Piper nigrum* /*Olea europaea* de 1/1/0,1/0,00325/0,6. La dose de cette composition était de 2% pendant les 4 premières semaines, puis de 2,7% durant la dernière semaine concomitante avec le traitement au DSS. Deux autres groupes ont reçu une dose de deux médicaments de référence pour le traitement des MICI (un corticostéroïde et un anticorps contre TNF-alpha), avant de commencer le traitement avec le DSS.

Pendant la période d'induction de l'inflammation avec le DSS, le poids des souris a été enregistré de façon journalière et des échantillons de fèces ont été pris pour calculer l'index d'activité de la maladie (Disease Activity Index, DAI). Cet index prend en compte trois paramètres : perte de poids, texture des fèces et présence de sang dans les fèces (voir **Tableau 4 :** Paramètres de calcul de l'index d'activité de la maladie) :

**[Tableau 4]**

| Points | Perte de poids | Texture des fèces | Présence de sang |
|---|---|---|---|
| 0 | <1% | Normal | Pas de sang |
| 1 | 1-5% | | |
| 2 | 5-10% | Mou et « collant » | Sang détecté avec un hémoculture |
| 3 | 10-15% | | |
| 4 | >15% | Diarrhée | Sang visible dans les fèces |

Le Tableau 5 présente le DAI calculé pour les différents groupes aux jours 1, 2 et 6 de la période d'induction de l'inflammation **(Tableau 5** : DAI aux jours 1, 2 et 6 de la période d'induction de l'inflammation. *p<0.05 vs anticorps contre TNF-alpha. ¥p<0.05 vs HFD+DSS).

**[Tableau 5]**

| Groupe | HFD+DSS | HFD+DSS+CX | HFD+DSS+Cortic | HFD+DSS+Ac |
|---|---|---|---|---|
| Jour 1 | 1.510.7 | 0±0^{∗} | 1.510.6 | 2.610.6 |
| Jour 2 | 2.710.7 | 1.410.5 | 2.7±0.3 | 2.310.6 |
| Jour 6 | 8.9±0.3 | 6.2±1^{¥} | 6.8±1.9 | 6.4±1.6 |

On constate que la composition X a diminué le DAI pendant toute la durée du traitement en comparaison au groupe non traité. De façon surprenante, au jour 1, le groupe traité avec la composition selon l'invention est le seul ayant un DAI de zéro, inférieur à celui observé dans les groupes traités avec les médicaments de référence. De plus, la composition selon l'invention a diminué de façon significative le DAI à la fin de l'induction par le DSS (jour 6) en comparaison au groupe non traité (de 8.9 à 6.2), tandis que les médicaments de référence n'atteignaient pas le seul de significativité.

Ainsi, la composition selon l'invention, en agissant de cette façon sur l'activité de la maladie, peut être utilisée dans le traitement des MICI.

## Revendications

1. Composition comprenant au moins :
- un extrait de Chrysanthellum, et
- un extrait d'artichaut, et
- un extrait de myrtillier, et
- un extrait d'au moins une matière première végétale comprenant de l'oleuropéine,
- et éventuellement de la pipérine synthétique et/ou un extrait d'au moins une matière première végétale comprenant de la pipérine,
pour une utilisation comme médicament ou produit de nutrition dans la prévention et/ou le traitement des maladies inflammatoires chroniques de l'intestin chez l'être humain ou l'animal.

2. Composition pour son utilisation selon la revendication 1, **caractérisée en ce que** l'extrait de Chrysanthellum est un extrait de *Chrysanthellum indicum.*

3. Composition pour son utilisation selon l'une des précédentes revendications, **caractérisée en ce que** l'extrait d'artichaut est choisi parmi les extraits de *Cyanara cardunculus,* les extraits de de *Cynara scolymus* et leurs mélanges.

4. Composition pour son utilisation selon l'une des précédentes revendications, **caractérisée en ce que** l'extrait de myrtillier est choisi parmi les extraits de *Vaccinium Myrtillus,* les extraits de *Vaccinium Angustifolium,* les extraits de *Vaccinium Corymbosum,* les extraits de *Vaccinium ashei Reade,* et leurs mélanges.

5. Composition pour son utilisation selon l'une des précédentes revendications, **caractérisée en ce que** l'extrait de matière première végétale comprenant de l'oleuropéine, est choisi parmi les extraits d'*Olea europeae,* les extraits de troène, les extraits d'Arganier, et leurs mélanges.

6. Composition pour son utilisation selon l'une des précédentes revendications, **caractérisée en ce que** l'extrait de matière première végétale comprenant de la pipérine est choisi parmi les extraits de *Piper,* les extraits de *Chalciporus piperatus* et leurs mélanges.

7. Composition pour son utilisation selon l'une des précédentes revendications, dans la prévention et/ou le traitement de la maladie de Crohn et/ou de la rectocolite hémorragique.

8. Composition pour une utilisation selon l'une des précédentes revendications, pour une réduction du score d'activité de la maladie prenant en compte 3 composantes majeures de la symptomatologie observée chez l'Homme : la perte de poids, le saignement et la texture des fèces.

9. Composition pour une utilisation selon l'une des précédentes revendications, **caractérisée** en qu'elle comprend au moins les molécules suivantes présentes dans les extraits :
- au moins une molécule choisie parmi l'apigenine-7-O-glucuronide, la chrysanthelline A, la chrysanthelline B, l'acide caféique, la lutéoline, la maritimétine, l'ériodictyol, l'isookanine, l'apigénine, la lutéoline-7-O-glucoside, la maritiméine, la maréine, l'ériodictyol-7-O-glucoside, la flavomaréine, l'apigénine-8-C-α-L-arabinoside-6-C-β-D-glucoside (shaftoside), l'apigénine-6,8-C-di-β-D-glucopyranoside (vicénine-2), et
- au moins une molécule choisie parmi un acide dicaféoylquinique, un acide sulfo-monocaféoylquinique, la lutéoline, la lutéoline-7-0-glucoside, la lutéoline-7-O-glucuronide, l'apigenine-7-O-glucoside, la cynaropicrine, et
- au moins une molécule choisie parmi un acide monocaféoylquinique, la delphinidine-3-galactoside, la delphinidine-3-glucoside, la cyanidine-3-galactoside, la delphinidine-3-arabinoside, la cyanidine-3-glucoside, la petunidine-3-galactoside, la cyanidine-3-arabinoside, la petunidine-3-glucoside, la péonidine-3-galactoside, la petunidine-3-arabinoside, la péonidine-3-glucoside, la malvidine-3-galactoside, la malvidine-3-glucoside, la malvidine-3-arabinoside,
- au moins de l'oleuropéine et éventuellement l'hydroxytyrosol, et
- éventuellement du verbascoside et/ou de la luteolin-7-o-glucuronide, et
- éventuellement de la pipérine.

10. Composition pour une utilisation selon l'une des précédentes revendications, **caractérisée en ce qu'**elle comprend au moins un acide dicaféoylquinique, du verbascoside, l'apigenine-7-O-glucuronide, de la luteolin-7-o-glucuronide, un acide monocaféoylquinique, l'oleuropéine et éventuellement de la pipérine.

11. Composition pour une utilisation selon l'une des précédentes revendications, **caractérisée en ce qu'**elle est exclusivement constituée par :
- un extrait de Chrysanthellum, et
- un extrait d'artichaut, et
- un extrait de myrtillier, et
- un extrait d'au moins une matière première végétale comprenant de l'oleuropéine,
- et éventuellement de la pipérine synthétique et/ou un extrait d'au moins une matière première végétale comprenant de la pipérine.

12. Composition pour une utilisation selon l'une des revendications 1 à 9, **caractérisée en ce qu'**elle comprend également au moins un élément supplémentaire ajouté en plus des extraits de plantes, ledit élément supplémentaire étant choisi parmi :
- les vitamines suivantes : B1, B2, B3, B5, B6, B8, B9, B12 C, A, D, E, K1 et K2 ;
- les composés suivants : acide obeticholique, acide corosolique, acides gras polyinsaturés de la famille des oméga 6 et/ou oméga 3, acide orotique, acide pangamique, acide para-amino-benzoïque, amygdaline, béta-glucanes, carnitine, diméthylglycine, imeglimine, isoflavones, L-arginine, oxytocine, pectine, pyridoxamine, resvératrol, viniférine, L-citrulline ;
- les oligo-éléments et minéraux suivants : arsenic, bore, calcium, cuivre, fer, fluor, iode, lithium, manganèse, magnésium, molybdène, nickel, phosphore, sélénium, vanadium, zinc ;
- les microconstituants à caractère non indispensable suivants : acide linolénique conjugué, acide lipoïque, caroténoïdes, carnitine, choline, coenzyme Q10, phytostérols, polyphénols de la famille des tannins et des lignanes, taurine ;
- les pré-biotiques;
- les probiotiques ;
- les anti-inflammatoires;
- les corticoïdes ;
- les dérivés aminosalicylés ;
- les anticorps monoclonaux ;
- les levures ;
- les champignons ;
- les produits dérivés d'insectes compatibles avec le secteur alimentaire et pharmaceutique ;
- la marijuana et le haschisch ;
- les agents d'enrobage ;
- les arômes;
- les acidifiants ;
- les antiagglomérants ;
- les épaississants ;
- les stabilisants ;
- les émulsifiants ;
- les agents de charge ;
- les excipients.

13. Composition pour une utilisation selon l'une des précédentes revendications, pour une administration par voie orale.

14. Composition pour une utilisation selon l'une des précédentes revendications, **caractérisée en ce qu'**elle se présente sous forme de poudre, de gel, d'émulsion, ou sous forme liquide, et en particulier sous forme de comprimés, capsules, gélules, sticks, sachets, ampoules, compte-gouttes ou sous forme injectable.

## Patentansprüche

1. Zusammensetzung, mindestens umfassend:
- einen Chrysanthellum-Extrakt, und
- einen Artischockenextrakt, und
- einen Heidelbeerextrakt, und
- einen Extrakt aus mindestens einem Pflanzenrohstoff, umfassend Oleuropein,
- und optional synthetisches Piperin und/oder einen Extrakt aus mindestens einem Pflanzenrohstoff, umfassend Piperin,
zur Verwendung als Arzneimittel oder Nahrungsprodukt bei der Prävention und/oder der Behandlung von chronischen entzündlichen Darmerkrankungen bei Menschen oder Tieren.

2. Zusammensetzung zur Verwendung nach Anspruch 1,
**dadurch gekennzeichnet, dass** der Chrysanthellum-Extrakt ein Extrakt aus *Chrysanthellum indicum* ist.

3. Zusammensetzung zur Verwendung nach einem der vorstehenden Ansprüche, **dadurch gekennzeichnet, dass** der Artischockenextrakt ausgewählt ist aus Extrakten aus *Cyanara cardunculus,* Extrakten aus *Cynara scolymus* und Mischungen davon.

4. Zusammensetzung zur Verwendung nach einem der vorstehenden Ansprüche, **dadurch gekennzeichnet, dass** der Heidelbeerextrakt ausgewählt ist aus Extrakten aus *Vaccinium Myrtillus,* Extrakten aus *Vaccinium Angustifolium,* Extrakten aus *Vaccinium Corymbosum,* Extrakten aus *Vaccinium ashei Reade,* und Mischungen davon.

5. Zusammensetzung zur Verwendung nach einem der vorstehenden Ansprüche, **dadurch gekennzeichnet, dass** der Extrakt aus Pflanzenrohstoff, umfassend Oleuropein, ausgewählt ist aus Extrakten aus *Olea europeae,* Extrakten aus Liguster, Extrakten des Arganbaumes und Mischungen davon.

6. Zusammensetzung zur Verwendung nach einem der vorstehenden Ansprüche, **dadurch gekennzeichnet, dass** der Extrakt aus Pflanzenrohstoff, umfassend Piperin, ausgewählt ist aus Extrakten aus *Piper,* Extrakten aus *Chalciporus piperatus* und Mischungen davon.

7. Zusammensetzung zur Verwendung nach einem der vorstehenden Ansprüche, bei der Prävention und/oder der Behandlung von Morbus Crohn und/oder Colitis ulcerosa.

8. Zusammensetzung zur Verwendung nach einem der vorstehenden Ansprüche, für eine Verringerung des Krankheitsaktivitäts-Scores unter Berücksichtigung von 3 Hauptkomponenten der bei dem Menschen beobachteten Symptomatik: Gewichtsverlust, Blutung und Beschaffenheit von Fäzes.

9. Zusammensetzung zur Verwendung nach einem der vorstehenden Ansprüche, **dadurch gekennzeichnet, dass** sie mindestens die folgenden in den Extrakten vorhandenen Moleküle umfasst:
- mindestens ein Molekül, ausgewählt aus Apigenin-7-O-glucuronid, Chrysanthellin A, Chrysanthellin B, Kaffeesäure, Luteolin, Maritimetin, Eriodictyol, Isookanin, Apigenin, Luteolin-7-O-glucosid, Maritimein, Marein, Eriodictyol-7-O-glucosid, Flavomarein, Apigenin-8-C-α-L-arabinosid-6-C-β-D-glucosid (Shaftosid), Apigenin-6,8-C-di-β-D-glucopyranosid (Vicenin-2), und
- mindestens ein Molekül, ausgewählt aus Dicaffeoylchinasäure, Sulfo-Monocafeoylchinasäure, Luteolin, Luteolin-7-O-glucosid, Luteolin-7-O-glucuronid, Apigenin-7-O-glucosid, Cynaropicrin, und
- mindestens ein Molekül, ausgewählt aus Monocaffeoylchinasäure, Delphinidin-3-galactosid, Delphinidin-3-glucosid, Cyanidin-3-galactosid, Delphinidin-3-arabinosid, Cyanidin-3-glucosid, Petunidin-3-galactosid, Cyanidin-3-arabinosid, Petunidin-3-glucosid, Peonidin-3-galactosid, Petunidin-3-arabinosid, Peonidin-3-glucosid, Malvidin-3-galactosid, Malvidin-3-glucosid, Malvidin-3-arabinosid,
- mindestens Oleuropein und optional Hydroxytyrosol, und
- optional Verbascosid und/oder Luteolin-7-O-glucuronid, und
- optional Piperin.

10. Zusammensetzung zur Verwendung nach einem der vorstehenden Ansprüche, **dadurch gekennzeichnet, dass** sie mindestens eine Dicaffeoylchinasäure, Verbascosid, Apigenin-7-O-glucuronid, Luteolin-7-O-glucuronid, eine Monocaffeoylchinasäure, Oleuropein und optional Piperin umfasst.

11. Zusammensetzung zur Verwendung nach einem der vorstehenden Ansprüche, **dadurch gekennzeichnet, dass** sie ausschließlich besteht aus:
- einem Chrysanthellum-Extrakt, und
- einem Artischockenextrakt, und
- einem Heidelbeerextrakt, und
- einem Extrakt aus mindestens einem Pflanzenrohstoff, umfassend Oleuropein,
- und optional synthetischem Piperin und/oder einem Extrakt aus mindestens einem Pflanzenrohstoff, der Piperin umfasst.

12. Zusammensetzung zur Verwendung nach einem der Ansprüche 1 bis 9, **dadurch gekennzeichnet, dass** sie auch mindestens ein zusätzliches Element umfasst, das zusätzlich zu den Pflanzenextrakten zugesetzt wird, wobei das zusätzliche Element ausgewählt ist aus:
- den folgenden Vitaminen: B1, B2, B3, B5, B6, B8, B9, B12, C, A, D, E, K1 und K2;
- den folgenden Verbindungen: Obeticholsäure, Corosolsäure, mehrfach ungesättigten Fettsäuren der Omega-6- und/oder Omega-3-Familie, Orotsäure, Pangaminsäure, Para-Aminobenzoesäure, Amygdalin, Beta-Glucanen, Carnitin, Dimethylglycin, Imeglimin, Isoflavonen, L-Arginin, Oxytocin, Pektin, Pyridoxamin, Resveratrol, Viniferine, L-Citrullin;
- den folgenden Oligo-Elementen und Mineralien: Arsen, Bor, Calcium, Kupfer, Eisen, Fluor, Jod, Lithium, Mangan, Magnesium, Molybdän, Nickel, Phosphor, Selen, Vanadium, Zink;
- den folgenden nicht essentiellen Mikrobestandteilen: konjugierter Linolensäure, Liponsäure, Carotinoiden, Carnitin, Cholin, Coenzym Q10, Phytosterolen, Polyphenolen aus der Familie der Tannine und Lignane, Taurin;
- Präbiotika;
- Probiotika;
- entzündungshemmenden Mitteln;
- Corticosteroiden;
- Aminosalicylderivaten;
- monoklonalen Antikörpern;
- Hefen;
- Pilzen;
- nahrungsmittel- und pharmazeutika-verträglichen Insektenderivaten;
- Marihuana und Haschisch;
- Überzugsmitteln;
- Aromastoffen;
- Säuerungsmitteln;
- Trennmitteln;
- Verdickungsmitteln;
- Stabilisatoren;
- Emulgatoren;
- Füllstoffen;
- Exzipienten.

13. Zusammensetzung zur Verwendung nach einem der vorstehenden Ansprüche für eine orale Verabreichung.

14. Zusammensetzung zur Verwendung nach einem der vorstehenden Ansprüche, **dadurch gekennzeichnet, dass** sie in Form von Pulver, Gel, Emulsion oder in flüssiger Form und insbesondere in Form von Tabletten, Kapseln, Gelatinekapseln, Sticks, Beuteln, Ampullen, Tropfflaschen oder in einer injizierbaren Form vorliegt.

## Claims

1. A composition comprising at least:
- an extract of Chrysanthellum, and
- an extract of artichoke, and
- an extract of blueberry, and
- an extract of at least one plant-derived raw material comprising oleuropein,
- and optionally synthetic piperine and/or an extract of at least one plant-derived raw material comprising piperine,
for use as a drug or nutritional product in the prevention and/or treatment of chronic inflammatory bowel disease in humans or animals.

2. The composition for its use according to claim 1, **characterized in that** the extract of Chrysanthellum is an extract of *Chrysanthellum indicum.*

3. The composition for its use according to one of the preceding claims, **characterized in that** the artichoke extract is selected from extracts of *Cyanara cardunculus,* extracts of *Cynara scolymus* and mixtures thereof.

4. The composition for its use according to one of the preceding claims, **characterized in that** the blueberry extract is selected from extracts of *Vaccinium Myrtillus,* extracts of *Vaccinium Angustifolium,* extracts of *Vaccinium Corymbosum,* extracts of *Vaccinium ashei Reade,* and mixtures thereof.

5. The composition for its use according to one of the preceding claims, **characterized in that** the plant-derived raw material extract comprising oleuropein is selected from extracts of *Olea europeae,* extracts of troene, extracts of argan, and mixtures thereof.

6. The composition for its use according to one of the preceding claims, **characterized in that** the plant raw material extract comprising piperine is selected from extracts of *Piper,* extracts of *Chalciporus piperatus* and mixtures thereof.

7. The composition for its use according to one of the preceding claims, in the prevention and/or treatment of Crohn's disease and/or ulcerative colitis.

8. The composition for its use according to one of the preceding claims, for a reduction in the disease activity score taking into account three major components of the observed symptomatology in humans: weight loss, bleeding and texture of the feces.

9. The composition for a use according to one of the preceding claims, **characterized in that** it comprises at least the following molecules present in the extracts:
- at least one molecule selected from apigenin-7-O-glucuronide, chrysanthelline A, chrysanthelline B, caffeic acid, luteolin, maritimetin, eriodictyol, isookanin, apigenin, luteolin-7-O-glucoside, maritimetin, marein, eriodictyol-7-O-glucoside, flavomarein, apigenin 8-C-α-L-arabinoside-6-C-β-D-glucoside (shaftoside), apigenin-6,8-C-di-β-D-glucopyranoside (vicenin-2), and
- at least one molecule selected from a dicaffeoylquinic acid, a sulfo-monocaffeoylquinic acid, luteolin, luteolin-7-0-glucoside, luteolin-7-O-glucuronide, apigenin-7-O-glucoside, cynaropicrin, and
- at least one molecule selected from a monocaffeoylquinic acid, delphinidin-3-galactoside, delphinidin-3-glucoside, cyanidine-3-galactoside, delphinidin-3-arabinoside, cyanidine-3-glucoside, petunidin-3-galactoside, cyanidine-3-arabinoside, petunidin-3-glucoside, peonidin-3-galactoside, petunidin-3-galactoside, peonidin-3-glucoside, malvidine-3-galactoside, malvidine-3-glucoside, malvidine-3-arabinoside,
- at least oleuropein and optionally hydroxytyrosol, and
- optionally verbascoside and/or luteolin-7-O-glucuronide, and
- optionally piperine.

10. The composition for a use according to one of the preceding claims, **characterized in that** it comprises at least one dicaffeoylquinic acid, verbascoside, apigenin-7-O-glucuronide, luteolin-7-O-glucuronide, a monocaffeoylquinic acid, oleuropein and optionally piperine.

11. The composition for a use according to one of the preceding claims, **characterized in that** it consists exclusively of:
- an extract of Chrysanthellum, and
- an extract of artichoke, and
- an extract of blueberry, and
- an extract of at least one plant-derived raw material comprising oleuropein,
- and optionally synthetic piperine and/or an extract of at least one plant-derived raw material comprising piperine,

12. The composition for a use according to one of claims 1 to 9, **characterized in that** it also comprises at least one additional element added in addition to the plant extracts, said additional element being selected from:
- the following vitamins: B1, B2, B3, B5, B6, B8, B9, B12 C, A, D, E, K1 and K2;
- the following compounds: obeticolic acid, corosolic acid, polyunsaturated fatty acids of the omega 6 and/or omega 3 family, orotic acid, pangamic acid, para-aminobenzoic acid, amygdalin, beta-glucans, carnitine, dimethylglycine, imeglimin, isoflavones, I-arginine, oxytocin, pectin, pyridoxamine, resveratrol, viniferin, L-citrulline,
- the following trace elements and minerals: arsenic, boron, calcium, copper, iron, fluorine, iodine, lithium, manganese, magnesium, molybdenum, nickel, phosphorus, selenium, vanadium, zinc;
- the following non-essential microconstituents: conjugated linolenic acid, lipoic acid, carotenoids, carnitine, choline, coenzyme Q10, phytosterols, polyphenols from the family of tannins and lignans, taurine;
- prebiotics;
- probiotics;
- anti-inflammatories
- corticosteroids;
- aminosalicylic derivatives;
- monoclonal antibodies;
- yeasts;
- fungi,
- insect-derived products compatible with the food and pharmaceutical sector;
- marijuana and hashish;
- coating agents;
- flavors;
- acidifiers;
- anticlumping agents;
- thickeners;
- stabilizers;
- emulsifiers;
- filler agents;
- excipients.

13. The composition for a use according to one of the preceding claims, for oral administration.

14. The composition for a use according to one of the preceding claims, **characterized in that** it is in the form of a powder, gel, emulsion, or in liquid form, and in particular in the form of tablets, capsules, gelcaps, sticks, pouches, ampoules, droppers or in injectable form.
